# EUROPEAN PATENT APPLICATION

(11) **EP 2 189 153 A2**
(43) Date of publication of application: **26.05.2010**
(21) Application number: 09252654.0
(22) Date of filing: 20.11.2009
(51) Int. Cl.: A61K 9/00, A61F 13/20

(54) **Article of manufacture used in contact with human body surfaces**

(30) Priority: 21.11.2008 US 116656 P; 19.12.2008 US 339340
(71) Applicant: McNeil-PPC, Inc., Skillman, NJ 08558 (US)
(72) Inventor: Linkel, Stephan M., Ewing, NJ 08628 (US)
(74) Representative: Kirsch, Susan Edith

(57) **Abstract**

The present invention provides an article designed to deliver the active ingredients to the various body cavities such vaginal, oral, nasal and rectal, further the article is designed in such a manner that it can be easily arranged and configured to fit into vaginal, oral, nasal, and rectal cavities.

## Description

### FIELD OF THE INVENTION

The present invention provides an article designed to deliver a composition to the vaginal, oral, nasal and/or rectal cavity and a method for manufacturing the article. The article exploits and/or protects the highly vascularized nature of the vaginal, oral, nasal and rectal mucosal tissue to deliver compositions to localized areas.

### BACKGROUND OF THE INVENTION

The vaginal, oral, nasal and rectal cavities provide a potential pathway for infection and disease to enter the body due to the copious blood supply in these regions and the lack of the stratum corneum that protects exposed surface skin.

The vaginal delivery route is known to be useful for the delivery of pharmaceutical agents that have their site of action within tissues or organs close to the vagina, in particular, for administration to the vaginal tissues.

Devices for the delivery of pharmaceutical agents and other compositions into the body cavities are known. Such devices are either of the type where a medicament is impregnated into the device, or of the type that carries an encapsulated medicament.

US Pat. No. 7,004,171 and published PCT Appl. No. WO 2003/070216 disclose a transvaginal drug delivery system comprising: (a) a deposition comprising an effective amount of the drug and, optionally, a wetting agent; and (b) a polymeric support on which the deposition is deposited.

US Pat. No. 7,341,737 discloses a tampon adapted to deliver a therapeutic agent, the tampon including a tampon body having a distal end; and a dosage form affixed to the distal end of the tampon body, wherein the dosage form includes a formulation including a therapeutic agent, and wherein the dosage form comprises a plurality of layers. Also, a method for manufacturing a medicated tampon adapted to deliver a therapeutic agent, the method including manufacturing a tampon body having a distal end; producing a dosage form having a plurality of layers, wherein the dosage form includes a formulation including a therapeutic agent; and affixing the dosage form to the distal end of the tampon body.

Brown-Skrobot, US Pat. No. 5,679,369, discloses additives to tampons to inhibit the production of toxic shock syndrome toxin-1. The additives generally are not liquid at or near room temperature, and therefore, they require a carrier material, such as isopropyl alcohol. This is representative of the delivery of an agent that acts to inhibit the growth of infections and/or disease in the body cavity.

In practice several problems are inherent in a process that attempts to introduce a composition into or onto an absorbent material or cover material of the article by coating, dipping, solidifying, or the like. Procedures such as these that may work in a laboratory setting may be precluded from application to an automated manufacturing process.

For example during manufacture of a tampon associated with active addititives, where careful dosing is one of requirements, the active additive and its carrier must be maintained in a solution that is both homogeneous and at a proper concentration and purity. These requirements are difficult to accomplish during normal operation, and are significantly more difficult to maintain when the tampon machine stops. In addition, tampons of different densities may absorb an applied active additive composition differently, resulting in variability in agent absorption into the tampons across different tampons.

Specifically, the requirement to provide constant agitation or mixing of the ingredients to the excipient and active compounds raises concerns as to how to keep the composition homogeneous when the manufacturing equipment stops during normal operation cycling. The use of inline mixers and recirculation of the heated therapeutic and excipient contained liquid compounds during machine stops may provide a method to keep the solution moving and mixed. However, because a machine could be stopped for several hours, the stability of some compound mixtures may be compromised by long durations at elevated temperatures, or by mechanical shear forces due to the continuous pumping of the recirculating solution also it runs into lot of machine maintenance related problems, to overcome this issue there is a need of an efficient method to incorporate a composition in to an article.

### SUMMARY OF THE INVENTION

We have found an improved method to incorporate compositions in articles of manufacture useful to deliver a composition to the vaginal, oral, nasal and/or rectal cavity.

We have discovered that associating the composition with at least one carrier that is then incorporated into the article overcomes many of the processing concerns of the prior art.

In one embodiment of the invention, an article suitable for insertion into a body cavity includes a first structure, a cover substantially containing the first structure, and a waxy composition associated with at least one carrier that is also substantially contained by the cover. The first structure is arranged and configured to fit into a body cavity. The cover is substantially liquid permeable material. The waxy composition comprises a compound or combination of compounds selected from the group consisting of;
i) monoesters of a polyhydric aliphatic alcohol and a fatty acid containing from eight to eighteen carbon atoms and wherein said monoester has at least one hydroxyl group associated with its aliphatic alcohol residue;
ii) diesters of a polyhydric aliphatic alcohol and a fatty acid containing from eight to eighteen carbon atoms and wherein said diester has at least one hydroxyl group associated with its aliphatic alcohol residue; and
iii) mixtures of said monoesters and diesters.

In an alternative embodiment of the invention a method of making an article suitable for insertion into a body cavity includes the steps of: (a) applying a waxy composition to at least one carrier; (b) providing a first structure arranged and configured to fit into a body cavity; and (c) substantially enclosing the first structure and the at least one carrier within an cover of material comprising a liquid permeable material. The waxy composition comprises a compound or combination of compounds selected from the group consisting of:
i) monoesters of a polyhydric aliphatic alcohol and a fatty acid containing from eight to eighteen carbon atoms and wherein said monoester has at least one hydroxyl group associated with its aliphatic alcohol residue;
ii) diesters of a polyhydric aliphatic alcohol and a fatty acid containing from eight to eighteen carbon atoms and wherein said diester has at least one hydroxyl group associated with its aliphatic alcohol residue; and
iii) mixtures of said monoesters and diesters.

### BRIEF DESCRIPTION OF THE DRAWING

Examples of embodiments of the present invention will now be described with reference to the drawings, in which:
Fig. 1 is a perspective view of an embodiment of an article in a rolled form.
Fig. 2 is a cross-sectional view of the article as shown in Fig. 1.
Fig. 3 is a perspective view of another embodiment of an article in a rolled form.
Fig. 4 is a cross-sectional view of the article as shown in Fig. 3.
Fig. 5 is a perspective view of an embodiment of an article in a folded form.
Fig. 6 is a cross-sectional view of the article as shown in Fig. 5.
Fig. 7 is a lateral cross-section of an article showing the construction of article.
Fig. 8 is a cross-sectional view of a yet another embodiment of an article in a rolled form.
Fig. 9 is a cross-sectional view of a yet another embodiment of an article in a rolled form.
Fig. 10 is a cross-sectional view of a yet another embodiment of an article in a folded form.
Fig. 11 is a perspective view of an embodiment of an article in a folded form showing the uniform application of the active ingredient associated carrier.
Fig. 12 is a perspective view of an embodiment of an article in a folded form showing the application of the active ingredient associated carrier at front-end portion.
Fig. 13 is a perspective view of yet another embodiment of an article having a substantially homogeneous first structure.
Fig. 14 is a cross-sectional view of the article as shown in Fig. 13.
Fig. 15 is schematic view of a process of making an article such as shown in Fig. 13.
Fig. 16 is schematic view of a process of making an article such as shown in Figs. 2 and 4.
Fig. 17 is schematic view of a process of making an article such as shown in Figs. 8-10.

### DETAILED DESCRIPTION OF THE INVENTION

The invention as described herein will be described for exemplary purposes using a tampon as an example of an article that is designed to fit into vaginal cavity to deliver a composition such as an active ingredient to the vagina. The invention, however, applies in a similar way to deliver compositions to or through the tissue of other body cavities like oral, nasal and rectal and hence invention should not be limited to the example described herein. The shape or dimensions of products may vary depending upon the anatomy of body cavity so as to give maximum benefits and comfort.

As used herein the term "first structure" may comprise absorbent and/or non-absorbent material. The absorbent material may consist of, but is not limited to, cellulose or cellulose derivative fibres, cotton, starch, rayon, sponge, woodpulp, polyolefin, polyester, polyamide, polyurethane,cross- linked carboxymethylcellulose, acrylic acid, methacrylic acid, 2-acrylamido-2-methyl propane sulphonic acid or a mixture thereof, or a hydrogel.

Waxy materials tend to be somewhat tacky, and difficulties arise in particular with regard to the handling of sheets coated or impregnated with such waxy materials during their production. The sheet and its waxy material tend to stick to machine parts and to foul the machinery with consequent process.

The non-absorbent material may be partially or completely non-absorbent or may have only a small degree of absorbency. More preferably the non-absorbent material is made up of, but is not limited to, polyester, polypropylene, polyethylene, aramid, nylon, acrylic, synthetic fibers, bicomponent or mixtures thereof.

As used herein the term "liquid-permeable" and variants thereof indicates that liquids, such as water or bodily fluids, are able to pass through a cover.

The liquid-permeable cover can be formed from woven and/or nonwoven materials having a porous substrate. The woven and nonwoven materials are well known in literature and would be readily understood by persons skilled in the art that any such liquid-permeable cover may be applied or used to deliver the present invention. The liquid-permeable cover can be formed from a non-porous materials having previously been processed into an apertured film. The aperturing processes are well known in literature and would be readily understood by persons skilled in the art such that any such liquid-permeable cover may be applied or used to deliver the present invention.

In one preferred embodiment, the composition is a waxy composition selected from the group consisting of;
i) monoesters of a polyhydric aliphatic alcohol and a fatty acid containing from eight to eighteen carbon atoms and wherein said monoester has at least one hydroxyl group associated with its aliphatic alcohol residue;
ii) diesters of a polyhydric aliphatic alcohol and a fatty acid containing from eight to eighteen carbon atoms and wherein said diester has at least one hydroxyl group associated with its aliphatic alcohol residue;
iii) mixtures of said monoesters and diesters.

The fatty acid portion of the said monoesters and diesters may be derived from caprylic, capric, lauric, myristic, palmitic and stearic acids, which are saturated fatty acids whose chain lengths, respectively, are C₈, C₁₀, C₁₂, C₁₄, C₁₆ and C₁₈. The fatty acid portion of the aforementioned monoesters and diesters may be derived as well from unsaturated fatty acids having carbon chain lengths also ranging from C₈ to C₁₈, one example of such unsaturated fatty acids being oleic acid. The preferred fatty acid for use in the practice of the present invention is lauric acid, a saturated fatty acid whose chemical formula is C₁₁ H₂₃ COOH.

As used in this specification and the appended claims, the term "aliphatic" has the meaning usually accorded it in organic chemistry, i.e. "aliphatic" refers to organic compounds characterized by straight--or branched--chain arrangement of the constituent carbon atoms.

As used in this specification and the appended claims, the term "polyhydric" refers to the presence in a chemical compound of at least two hydroxyl (OH) groups. Thus, a polyhydric aliphatic alcohol is one which has at least two hydroxyl groups and in which the carbon backbone is either straight or branched.

Polyhydric alcohols suitable for forming monoesters and/or diesters for use in the practice of the present invention are 1,2-ethanediol; 1,2,3-propanetriol (glycerol); 1,3-propanediol; 1,4-butanediol; 1,2,4-butanetriol and the like. The preferred polyhydric aliphatic alcohol for forming monoesters and diesters for use in the practice of the present invention is 1,2,3-propanetriol (commonly called glycerol) whose formula is HOCH₂ CH(OH)CH₂ OH.

The monoester of glycerol and one of the designated fatty acids may be used in the practice of the present invention because that ester will have two hydroxyl groups associated therewith which are derived from the glycerol. The diester of glycerol and one of the designated fatty acids may also be used because that ester will have one hydroxyl group associated therewith which is derived from the aliphatic alcohol glycerol. Indeed, as will be seen hereinafter, blends of glycerol monolaurate and glycerol dilaurate have been found to be useful in the practice of the present invention. Preferred esters for use in the practice of the present invention are glyceryl monolaurate, glyceryl dilaurate and mixtures thereof.

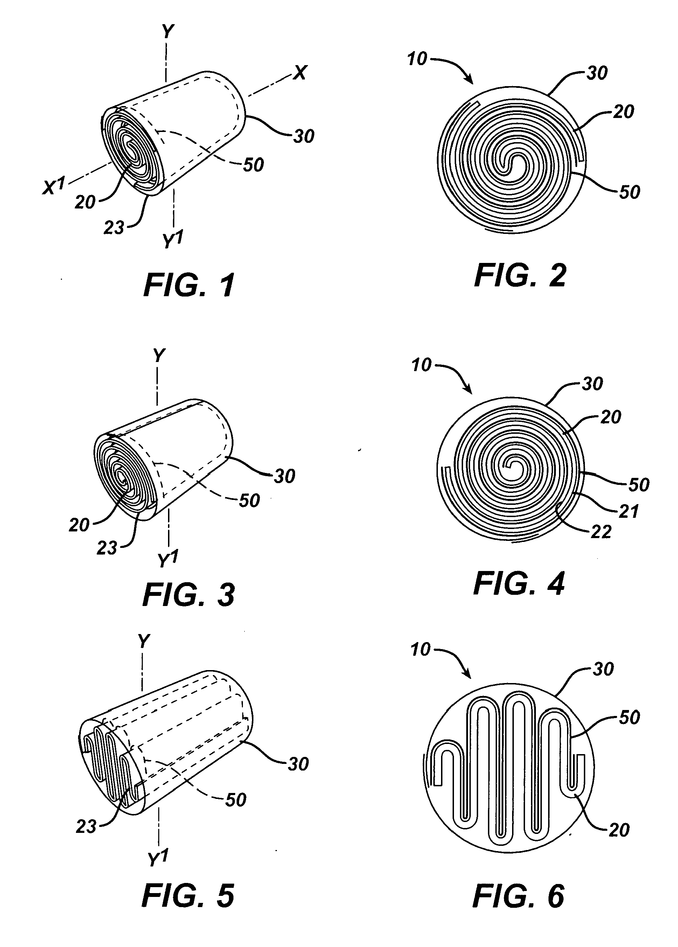

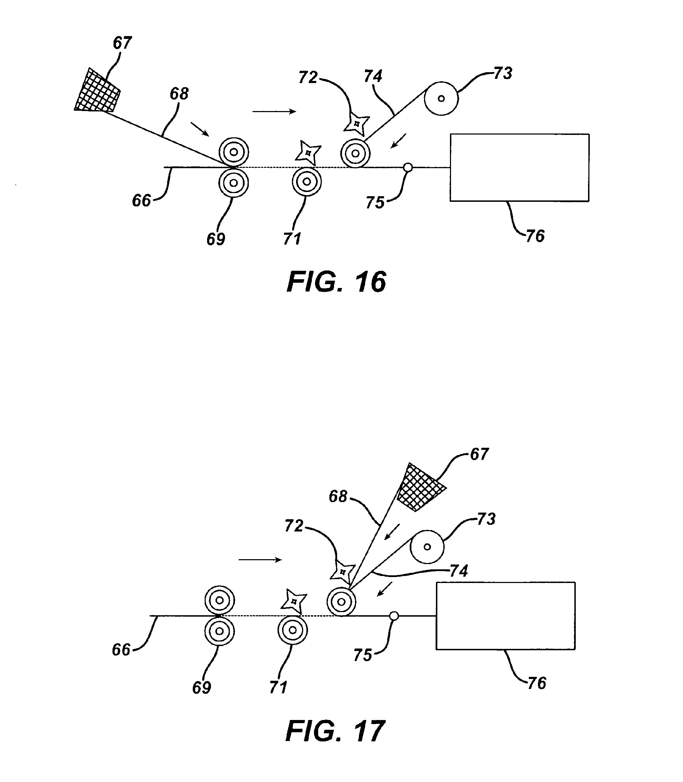

Referring to the drawings in greater detail, Figs. 1-15 illustrate an article 10 of the present invention which is designed to deliver active ingredient through vaginal, oral, nasal and rectal cavity and Figs. 16 and 17 illustrate method of making the same. The article 10, however can be in many other forms, and is not limited to a structure having the particular configuration as shown in these Figures.

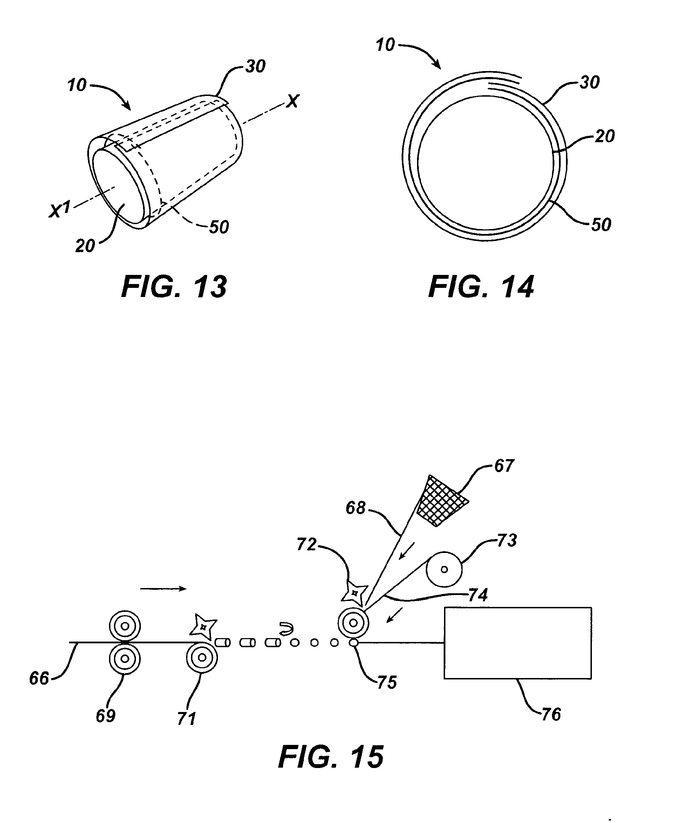

As seen in the Figures, article 10, generally includes a first structure 20, a cover 30 and an active ingredient associated with carrier 50. Article 10, can have a front-end portion 13 for insertion of the article 10 into body cavity, a center portion 14, and a rear-end portion 15 for gripping or withdrawing the article 10. First structure 20, can be in the form of a roll, e.g., wound about a center portion of a fibrous sliver as shown in Fig. 1, 2, and 8 (and described more fully in Friese, US Pat. No. 4,816,100, the contents of which are hereby incorporated by reference), or wound in a convolutedly rolled configuration as shown in Fig. 3, 4, and 9, or in a gathered configuration as shown in Fig. 5, 6, and 10. The first structure 20 can also be a substantially homogeneous (either loose or relatively rigid) structure, as shown in Figs. 13 and 14.

First structure 20, comprises an outer surface 21 and an inner surface 22, with an front end portion 23 aligned with front-end portion 13, center portion 24 aligned with center portion 14, and rear portion 25 aligned with rear-end portion 15 of the article 10.

First structure 20 is substantially enclosed with a cover 30, comprising an inner surface 32 facing the outer surface 21 of the first structure 20, and an outer surface 31 facing the body cavity. An active ingredient 40 associated with carrier 50, is substantially positioned within the article.

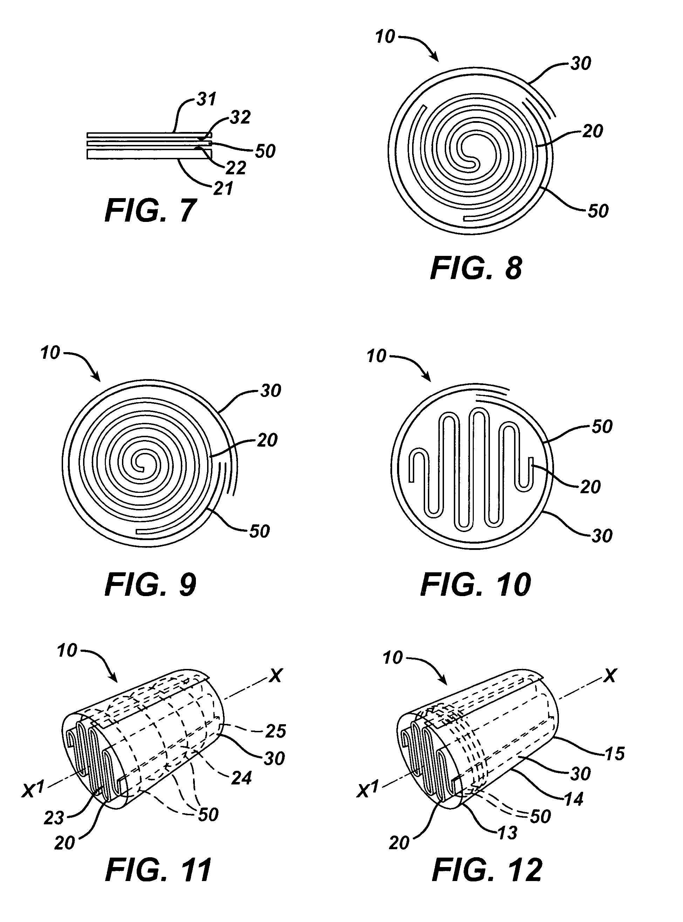

As can be seen in Figs. 8-12, the carrier 50 is positioned generally between the cover 30 and the first structure 20. In an alternate embodiment of invention shown in Figs. 1-6, shows the carrier 50 embedded within the first structure 20.

As best seen in Fig. 11 and 12 there can be at least one carrier element 50, and the location of the carriers can be optimized for a particular purpose. In particular, the carrier 50 can be concentrated at any of end or carriers can be uniformly placed over the entire article 10. The carrier 50, can be in the form of ribbon-like strip or mono-filament or multi-filament cord .

As seen figures article 10 of the present invention is an elongated fibrous structure comprising a cylindrical, spherical or ellipsoid shape, for simplicity in understanding the dimension of article 10, Fig.1 shows a longitudinal axis X-X' and lateral axis Y-Y' shaped around the article 10.

In general, the method of making an article of the present invention is typically divided in two steps;

Step 1: The selected composition to be applied may be dissolved in a suitable diluent to form a liquid mixture that is uniformly applied, to a carrier. The diluent may be driven off (e.g., a volatile solvent), or if desired may remain as part of the coating composition. The carrier coated with the selected composition may be rolled up or otherwise stored until desired for processing into an article of manufacture.

Step 2: In one embodiment, illustrated in Fig. 16, a supply of material useful to form the first structure 20, e.g., an absorbent fibrous sliver 66 is provided and combined with the coated carrier 68 from a carrier supply 67. The sliver 66 and coated carrier 68 are drawn through pull rolls 69, and then passed through a cutting station 71. The resulting combination of sliver 66 and coated carrier 68 is combined with a length of cover material 74. The cover material length 74 is provided by roller 73 and a cover cutting station 72. The sliver, carrier, and cover are rolled up or folded as per the requirement in a former, e.g., winder 75. The thus formed article can then be transferred for further processing such as in a tampon press 76.

In an alternate embodiment shown in Fig. 17, the coated carrier 68 is provided, not with the sliver as with the process shown in Fig. 16, but with the cover material 74. The cover material 74 and carrier 68 are then wrapped about the outer portions of the sliver 66 at the former 75. Again, the resulting article can then be transferred for further processing such as in a tampon press 76.

## Claims

1. An article suitable for insertion into a body cavity comprising:
a) a first structure;
b) a cover comprising a liquid permeable material substantially containing the first structure; and
c) a waxy composition associated with at least one carrier, which is substantially contained by the cover, the waxy composition comprising a compound or combination of compounds selected from the group consisting of;
i) monoesters of a polyhydric aliphatic alcohol and a fatty acid containing from eight to eighteen carbon atoms and wherein said monoester has at least one hydroxyl group associated with its aliphatic alcohol residue;
ii) diesters of a polyhydric aliphatic alcohol and a fatty acid containing from eight to eighteen carbon atoms and wherein said diester has at least one hydroxyl group associated with its aliphatic alcohol residue; and
iii) mixtures of said monoesters and diesters;
wherein the first structure is arranged and configured to fit into a body cavity.

2. The article of claim 1, wherein the first structure is absorbent, and/or substantially non-absorbant.

3. The article of claim 1 or 2, wherein said at least one carrier is positioned between an outer surface of the first structure and an inner surface of the cover.

4. The article of claim 3, wherein said at least one carrier is substantially wrapped around the outer surface of the first structure.

5. The article of claim 3, wherein said at least one carrier is attached to the cover.

6. The article of claim 1 or 2, wherein said at least one carrier is substantially contained within the first structure.

7. The article of any preceding claim, wherein said at least one carrier comprises an element having a substantially ribbon-like structure; or a plurality of substantially ribbon-like structures; or a fibrous cord, wherein the fibrous cord is a monofilament cord or a multifilament cord.

8. The article of any preceding claim, wherein said at least one carrier comprises an apertured film structure.

9. The article of any preceding claim, wherein said cover comprises an apertured film and/or a nonwoven fabric.

10. The article of any preceding claim, wherein said fatty acid is lauric acid.

11. The article of any preceding claim, wherein said polyhydric alcohol is glycerol.

12. The article of any preceding claim, wherein said compound comprises glyceryl monolaurate.

13. A method of making an article suitable for insertion into a body cavity comprising the steps of:
a) applying a waxy composition to at least one carrier, wherein the waxy composition comprises a compound or combination of compounds selected from the group consisting of:
i) monoesters of a polyhydric aliphatic alcohol and a fatty acid containing from eight to eighteen carbon atoms and wherein said monoester has at least one hydroxyl group associated with its aliphatic alcohol residue;
ii) diesters of a polyhydric aliphatic alcohol and a fatty acid containing from eight to eighteen carbon atoms and wherein said diester has at least one hydroxyl group associated with its aliphatic alcohol residue; and
iii) mixtures of said monoesters and diesters;
b) providing a first structure arranged and configured to fit into a body cavity; and
c) substantially enclosing the first structure and the at least one carrier within an cover of material comprising a liquid permeable material.

14. The method of claim 13, wherein said at least one carrier is attached to the cover of material.

15. The method of claim 13 or 14, wherein said at least one carrier is substantially wrapped around an outer surface of the first structure.

16. The method of claim 13, wherein said at least one carrier is substantially contained within the first structure. ,
